# EUROPEAN PATENT SPECIFICATION

(11) **EP 3 556 853 B1**
(45) Date of publication and mention of the grant of the patent: **22.09.2021**
(21) Application number: 16923967.0
(22) Date of filing: 13.12.2016
(51) Int. Cl.: B01L 3/00, C12N 15/09, C12M 1/00, G01N 37/00, C12Q 1/68, G01N 33/543

(54) **MICROCHIP**
MIKROCHIP
MICROPUCE

(43) Date of publication of application: 23.10.2019
(73) Proprietor: Eiken Kagaku Kabushiki Kaisha, Taito-ku Tokyo 110-8408 (JP)
(72) Inventor: WATANABE, Hidetoshi, Tochigi 329-0114 (JP)
(74) Representative: Boult Wade Tennant LLP
(86) International application number: PCT/JP2016/087025
(87) International publication number: WO 2018/109829

(56) References cited:
- EP-A1- 2 812 116
- WO-A1-2013/132891
- WO-A1-2013/157667
- WO-A1-2014/010299
- JP-A- 2008 211 967
- JP-A- 2013 101 154
- JP-A- 2014 132 228
- JP-A- 2016 211 871
- JP-B2- S5 218 443
- US-A1- 2013 153 424

## Description

### Technical Field

The present invention relates to a microchip in which an injected sample solution is reacted with a reagent.

### Background Art

In recent years, a nucleic acid amplification device using a polymerase chain reaction (PCR) method, a loop-mediated isothermal amplification (LAMP) method or the like has been used as a genetic test. Further, in the genetic test using the nucleic acid amplification device, a technique for amplifying a nucleic acid in a microchip has been developed (see, for example, Patent Literature 1).

The microchip has a three-layer structure of a first plate-shaped part, a second plate-shaped part and a third plate-shaped part. The first plate-shaped part and the third plate-shaped part have gas impermeability, and the second plate-shaped part has self-sealing property. Further, the first plate-shaped part and the third plate-shaped part are stacked on both sides of the second plate-shaped part, and a reaction space depressurized with respect to the atmospheric pressure is formed between the first plate-shaped part and the second plate-shaped part. The reaction space is divided into an injection space which is punctured by a needle and into which a sample solution is injected, a plurality of reagent sealing spaces in which a reagent for amplifying the nucleic acid is sealed, and a flow path for connecting the injection space and each reagent sealing space.

Further, when a needle (a hollow needle) injecting the sample solution is punctured into the second plate-shaped part, the sample solution is introduced from the needle into the injection space by the negative pressure of the reaction space, and is further introduced into each reagent sealing space through the flow path. Further, the nucleic acid contained in the sample solution is mixed with the reagent in each reagent sealing space and amplified by incubation at a predetermined temperature which is increased.

EP 2812116 A1 describes a microchip configured to contain a sample solution for analysis.

US 2013/153424 A1 describes a microchip comprising an introduction area having a pressure negative to atmospheric pressure and into which a liquid is injected by puncturing, and a degassing area.

JP 2013101154 A also describes a microchip configured to contain a sample solution for analysis.

### Citation List

### Patent Literature

Patent Literature 1: Japanese Patent No. 5218443

### Summary of Invention

### Technical Problem

In such a microchip, when the needle punctured into the second plate-shaped part deflects, there is a possibility that a gap is generated between the needle and the second plate-shaped part, and air may flow into the injection space from the gap. In addition, there is a possibility that a crack may extend from a puncture hole formed by puncturing of the needle, and air may flow into the injection space from the extended crack. The sample solution is introduced into the reagent sealing space through the flow path by negative pressure. For this reason, when air flows into the injection space from such a gap or crack, the sample solution does not reach the reagent sealing space, which causes a problem in the nucleic acid amplification reaction. Since such a problem is more likely to occur as the second plate-shaped part becomes thinner, it is preferable that the second plate-shaped part be as thick as possible.

On the other hand, as the microchip becomes thicker, the responsiveness worsens when heated or cooled, and it is difficult to obtain an overall uniform heat distribution. In addition, when the temperature rising rates of each reagent sealing space are different, the amplification rates of nucleic acid in each reagent sealing space are also different. For this reason, if the microchip is too thick, it becomes difficult to carry out a high-precision inspection.

Therefore, it is conceivable to suppress the non-uniform heat distribution and to suppress the inflow of air, by thickening the second plate-shaped part, while suppressing the thickness of the entire microchip. However, since the microchip of the related art has a three-layer structure in which the first plate-shaped part and the third plate-shaped part are stacked on both sides of the second plate-shaped part, there is a limit to thickening the second plate-shaped part, while suppressing the thickness of the entire microchip.

Thus, an object of this invention is to provide a microchip which can suppress inflow of air by puncturing of the needle, while suppressing the non-uniform heat distribution.

### Solution to Problem

The invention is defined in the claims and provides a microchip (1, 1A, 1B, 1C) equipped with a chip main body (2) in which a reaction space (3) depressurized with respect to an atmospheric pressure is formed,
wherein the chip main body (2) has a two-layer structure of a first plate-shaped part (7) having gas impermeability, and a second plate-shaped part (8) having a self-sealing property, which is laminated on one surface of the first plate-shaped part (7), and
the reaction space is formed between the first plate-shaped part (7) and the second plate-shaped part (8),
wherein the microchip (1, 1A, 1B, 1C) further comprises a reinforcing film (10A, 10B, 10C) stuck to a surface of the second plate-shaped part (8) on a side opposite to the first plate-shaped part (7),
wherein the reaction space (3) has an injection space (3a) for puncturing and injecting a sample solution, and
the reinforcing film (10A, 10B, 10C) is stuck to a position facing the injection space (3a),
wherein the second plate-shape part is made of an elastic material, which allows for self-sealing of any holes made by puncturing or the like due to its own elastic deformation.

In all aspects, the microchip includes a reinforcing film stuck to a surface of the second plate-shaped part on a side opposite to the first plate-shaped part. In the microchip, since the reinforcing film is stuck to the surface of the second plate-shaped part on the side opposite to the first plate-shaped part, deformation of the second plate-shaped part is suppressed at the position to which the reinforcing film is stuck. Therefore, the deflection of the needle punctured in the second plate-shaped part can be suppressed, and the extension of the crack from the puncture hole can be suppressed. This makes it possible to suppress the inflow of air due to the puncturing of the needle.

In all aspects, the reaction space has an injection space into which a sample solution is punctured and injected, and the reinforcing film is stuck to a position facing the injection space. In this microchip, since the reinforcing film is stuck to the position facing the injection space, when the needle is punctured, it is possible to efficiently suppress air from flowing in the reaction space.

In an aspect, a checking space independent of the reaction space may be formed between the first plate-shaped part and the second plate-shaped part, and the reinforcing film may be stuck to a position facing the checking space. In this microchip, since the reinforcing film is stuck to the position facing the checking space, when inserting the needle into the checking space, it is possible to efficiently suppress air from flowing into the checking space.

In an aspect, the reinforcing film may be integrally stuck to the position facing the injection space and the position facing the checking space. In this microchip, since the reinforcing film is integrally stuck to the position facing the injection space and the position facing the checking space, the workability at the time of sticking the reinforcing film to both positions is improved.

In an aspect, the reinforcing film may be stuck to the entire surface of the second plate-shaped part on the side opposite to the first plate-shaped part. In this microchip, since the reinforcing film is stuck to the entire surface of the second plate-shaped part on the side opposite to the first plate-shaped part, it is possible to suppress the air from transmitting from the second plate-shaped part side to the reaction space and the checking space. Moreover, since the reinforcing film is also stuck to a surplus position that does not face the reaction space and the checking space of the second plate-shaped part, character and the code indicating identification information or the like of the microchip can be printed on the portion of the reinforcing film stuck to the surplus position. This makes it possible to reduce the work of sticking a label to the microchip separately.

In an aspect, the reinforcing film may be stuck to 50% or more of the surface of the second plate-shaped part on the side opposite to the first plate-shaped part. In this microchip, since the reinforcing film is stuck to 50% or more of the surface of the second plate-shaped part on the side opposite to the first plate-shaped part, it is possible to effectively suppress the air from transmitting from the second plate-shaped part side to the reaction space. Moreover, since the reinforcing film is also stuck to the surplus position that does not face the reaction space and the checking space of the second plate-shaped part, character and the code indicating identification information or the like of the microchip can be printed on the portion of the reinforcing film stuck to the surplus position. This makes it possible to reduce the work of sticking a label to the microchip separately.

In an aspect, the reinforcing film may include a base material, and an adhesive part for sticking the base material to the second plate-shaped part, a material of the base material may be polyurethane or polyester, and a thickness of the base material may be 10 µm or more and 30 µm or less. In this microchip, high stretchability can be obtained by setting the material and thickness of the base material forming the reinforcing film as above. For this reason, it is possible to suppress the puncture hole generated in the second plate-shaped part from spreading, and to suppress extension of a crack from the puncture hole.

In an aspect, the reinforcing film may include a base material, and an adhesive part for sticking the base material to the second plate-shaped part, a material of the base material may be polypropylene or polyester, and a thickness of the base material may be 20 µm or more and 100 µm or less. In this microchip, by setting the material and thickness of the base material forming the reinforcing film as above, appropriate rigidity can be obtained. For this reason, the workability of sticking to the second plate-shaped part is improved. Furthermore, it is possible to suppress the puncture hole generated in the second plate-shaped part from spreading, and to suppress extension of a crack from the puncture hole.

In an aspect, the reinforcing film may have a colored layer in which the side of the coloured layer that faces the second plate-shaped part is colored in dark color. In this microchip, by forming the colored layer on the reinforcing film, it is possible to suppress stray light when detecting amplification of nucleic acid.

### Advantageous Effects of Invention

According to this invention, it is possible to suppress the inflow of air by puncturing of the needle, while suppressing the uneven heat distribution.

### Brief Description of Drawings

FIG. 1 is a plan view illustrating a microchip according to a first example, not forming part of the invention.
FIG. 2 is a schematic cross-sectional view taken along line II-II illustrated in FIG. 1.
FIG. 3 is a schematic cross-sectional view taken along line III-III illustrated in FIG. 1.
FIG. 4 is a schematic cross-sectional view illustrating a state in which a sample solution is punctured and injected into a reaction space.
FIG. 5 is a schematic cross-sectional view illustrating a state in which the sample solution is punctured and injected into a checking space.
FIG. 6 is a schematic cross-sectional view illustrating a state in which a needle is punctured in a microchip of a comparative example.
FIG. 7 is a schematic cross-sectional view illustrating a state in which the needle is punctured in the microchip of the first example.
FIG. 8 is a plan view illustrating a microchip of a first embodiment of the invention.
FIG. 9 is a schematic cross-sectional view taken along line IX-IX illustrated in FIG. 8.
FIG. 10 is a schematic cross-sectional view of a reinforcing film.
FIG. 11 is a schematic cross-sectional view illustrating a state in which the sample solution is punctured and injected into the reaction space.
FIG. 12 is a schematic cross-sectional view illustrating a state in which the needle is punctured in the microchip of the first embodiment.
FIG. 13 is a plan view illustrating a microchip of a second embodiment of the invention.
FIG. 14 is a plan view illustrating a microchip of a third embodiment.
FIG. 15 is a schematic cross-sectional view illustrating the configuration of the reinforcing film.
FIG. 16 is a bottom view illustrating a second plate-shaped part side of the reinforcing film.
FIG. 17 is a bottom view illustrating the second plate-shaped part side of the reinforcing film.

### Description of Embodiments and Examples

Hereinafter, embodiments of the microchip according to the present invention and other examples will be described with reference to the drawings. The microchip according to the embodiment is a microchip into which a sample solution containing a nucleic acid is punctured and injected by a needle (a hollow needle) in order to amplify the nucleic acid and perform a genetic test. The same or corresponding elements in the drawings will be denoted by the same reference numerals and repeated description will not be provided.

### [First Example]

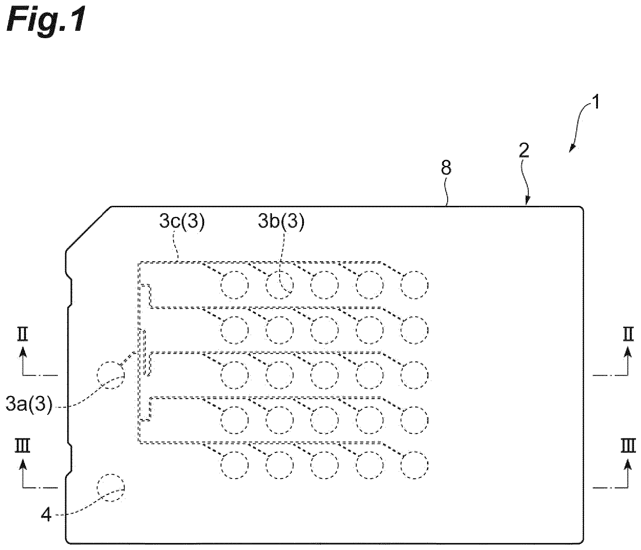

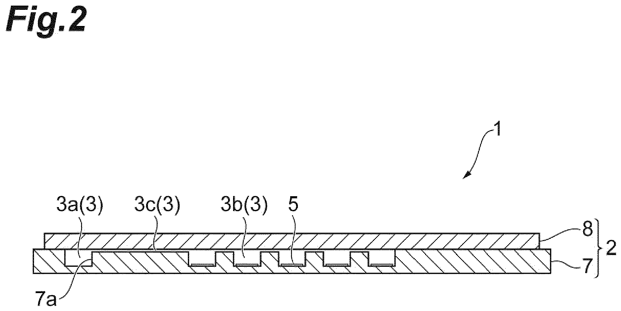

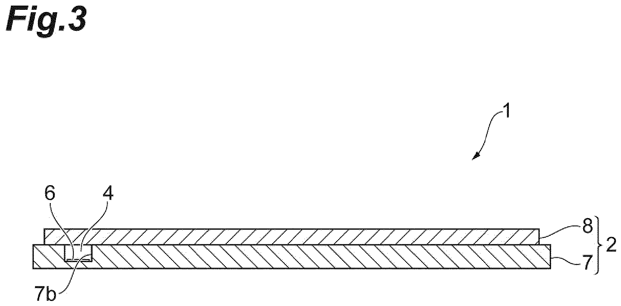

FIG. 1 is a plan view illustrating a microchip of a first example, not forming part of the invention. FIG. 2 is a schematic cross-sectional view taken along line II-II illustrated in FIG. 1. FIG. 3 is a schematic cross-sectional view taken along line III-III illustrated in FIG. 1. As illustrated in FIGS. 1 to 3, in a microchip 1 of this example, a reaction space 3 and a checking space 4 which are independent of each other are formed in a transparent chip main body 2. In the present example, the term "transparent" is meant to also include semi-transparency to the extent that it has optical transparency in addition to complete transparency.

The reaction space 3 is a sealed space formed inside the microchip 1. The reaction space 3 is depressurized with respect to the atmospheric pressure, and can be set to, for example, 1/100 atm or less. A reagent 5 for reacting with the sample solution to be injected is sealed in the reaction space 3. In the present example, a reagent corresponding to the nucleic acid contained in the sample solution is used as the reagent 5.

The reaction space 3 has an injection space 3a, a plurality of reagent sealing spaces 3b, and a flow path 3c.

The injection space 3a is a space into which the sample solution is punctured and injected, and is also called a cell or a well. A space shape of the injection space 3a is not particularly limited, but can be, for example, a substantially cylindrical space.

The reagent sealing space 3b is a space in which the reagent 5 is sealed, and is also called a cell or a well. The number and arrangement of the reagent sealing spaces 3b are not particularly limited, but for example, it is possible to adopt a total of 25 rows of 5 rows in a vertical direction (up-down direction in FIG. 1) and 5 rows in a horizontal direction (left-right direction in FIG. 1). In a case where there is a plurality of reagent sealing spaces 3b, it is preferable to seal the reagents for causing different nucleic acids to undergo amplification reactions in each of the reagent sealing spaces 3b. As a result, the nucleic acid can be subjected to amplification reaction only in the reagent sealing space 3b in which the reagent 5 corresponding to the nucleic acid contained in the sample solution punctured and injected into the injection space 3a is sealed. The space shape of the reagent sealing space 3b is not particularly limited, but can be, for example, a substantially cylindrical space.

The flow path 3c is a flow path through which the injection space 3a and each reagent sealing space 3b communicate with each other, and is also called a route or a channel. Specifically, one flow path 3c extends from the injection space 3a, and the flow path 3c branches into the number of the reagent sealing space 3b on the way from the injection space 3a to the reagent sealing space 3b, and each of branched flow paths 3c is connected to each reagent sealing space 3b. Although the cross-sectional shape of the flow path 3c is not particularly limited, it may be, for example, semicircular.

The checking space 4 is a sealed space formed inside the microchip 1. The checking space 4 is decompressed with respect to the atmospheric pressure, and can be set, for example, 1/100 atm or less. A discoloring agent 6 that changes color when the sample solution is mixed is sealed in the checking space 4. The checking space 4 is a space in which the discoloring agent 6 is sealed, and is also called a cell or a well. A space shape of the checking space 4 is not particularly limited, but can be, for example, a substantially cylindrical space.

The chip main body 2 is formed in a substantially thin rectangular plate shape. The chip main body 2 has a two-layer structure of a first plate-shaped part 7, and a second plate-shaped part 8 stacked on one surface of the first plate-shaped part 7. The first plate-shaped part 7 and the second plate-shaped part 8 are joined in a state being superimposed on each other.

The first plate-shaped part 7 is formed in a substantially thin rectangular plate shape. A reaction space recessed part 7a and a checking space recessed part 7b are formed on the surface of the first plate-shaped part 7 on the second plate-shaped part 8 side. The reaction space recessed part 7a is a recessed part for forming the reaction space 3. The shape of the reaction space recessed part 7a corresponds to the shapes of each of the injection space 3a, the plurality of reagent sealing spaces 3b, and the flow path 3c. The checking space recessed part 7b is a recessed part for forming the checking space 4. The shape of the checking space recessed part 7b corresponds to the shape of the checking space 4.

The first plate-shaped part 7 has gas impermeability. Although a material of the first plate-shaped part 7 is not specifically limited, glass, a synthetic resin, and the like which have gas impermeability can be used. Examples of the synthetic resin include polymethyl methacrylate (PMMA: acrylic resin), polycarbonate (PC), polystyrene (PS), polyethylene terephthalate (PET), COP, cyclic polyolefin (COC), and the like.

The second plate-shaped part 8 is formed in a substantially thin rectangular plate shape. The second plate-shaped part 8 covers at least the reaction space recessed part 7a and the checking space recessed part 7b. In this case, the second plate-shaped part 8 preferably covers the entire surface of the first plate-shaped part 7. The second plate-shaped part 8 covers the reaction space recessed part 7a, thereby forming the injection space 3a, the plurality of reagent sealing spaces 3b and the flow path 3c of the reaction space 3 between the first plate-shaped part 7 and the second plate-shaped part 8. Moreover, the second plate-shaped part 8 covers the checking space recessed part 7b, thereby forming the checking space 4 between the first plate-shaped part 7 and the second plate-shaped part 8.

The second plate-shaped part 8 has a self-sealing property. The self-sealing property refers to a property in which, even if a hole is made by puncturing or the like, the hole is naturally sealed by a restoring force due to its own elastic deformation. Examples of the elastic material used for the second plate-shaped part 8 can include silicone elastomers, acrylic elastomers, urethane elastomers, fluorine elastomers and the like. The second plate-shaped part preferably further has gas permeability, and can adopt polydimethylsiloxane (PDMS) which is an elastic material having self-sealing property and gas permeability.

The microchip 1 configured as described above can be manufactured as follows. First, a transparent dielectric film such as SiO₂, Al₂O₃ and TiO is coated on the surface of the first plate-shaped part 7 to be joined to the second plate-shaped part 8. The coating of the transparent dielectric film can be performed, for example, by sputtering or vacuum evaporation. Next, the reagent 5 is dropped to the recessed part corresponding to the reagent sealing space 3b of the reaction space recessed part 7a, the discoloring agent 6 is dropped to the checking space recessed part 7b, and the reagent 5 and the discoloring agent 6 are vacuum-freeze dried. Next, the surface of the first plate-shaped part 7 to be joined to the second plate-shaped part 8, and the surface of the second plate-shaped part 8 to be joined to the first plate-shaped part 7 are subjected to plasma cleaning. Next, the first plate-shaped part 7 and the second plate-shaped part 8 are superposed under a depressurized atmosphere (a state of being depressurized with respect to the atmospheric pressure). Therefore, the first plate-shaped part 7 and the second plate-shaped part 8 are joined, and the reaction space 3 and the checking space 4 depressurized with respect to the atmospheric pressure are formed between the first plate-shaped part 7 and the second plate-shaped part 8. Further, the reaction space 3 and the checking space 4 have substantially the same pressure.

Next, a method of genetic test using the microchip 1 will be described.

In the genetic test, after passing through a checking step (S1) of puncturing and injecting the sample solution into the checking space 4, a reaction step (S2) of puncturing and injecting the sample solution into the reaction space 3 is performed.

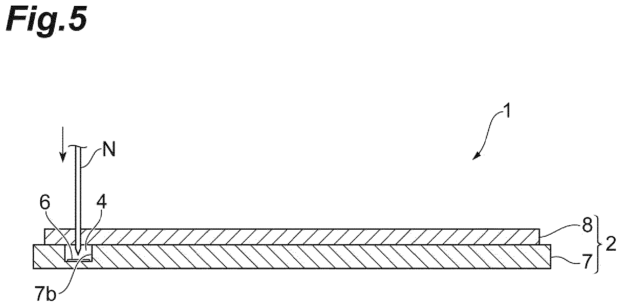

FIG. 5 is a schematic cross-sectional view illustrating a state in which the sample solution is punctured and injected into the checking space. As illustrated in FIG. 5, in the checking step (S1), the sample solution is punctured and injected into the checking space 4, using a needle N connected to a container (not illustrated) filled with the sample solution. Specifically, the needle N is punctured into the second plate-shaped part 8, and a distal end portion of the needle N is inserted into the checking space 4.

Then, when the depressurized state of the checking space 4 is sufficiently maintained, the sample solution is sucked from the needle N to the checking space 4 by the negative pressure of the checking space 4 and spreads over the entire checking space 4. Further, the discoloring agent 6 is mixed with the sample solution and changes color. Therefore, in the used microchip 1, the discoloring agent 6 in the checking space 4 is in a discolored state. On the other hand, when the depressurized state of the checking space 4 is not sufficiently maintained, since the negative pressure of the checking space 4 becomes weak, the force for sucking the sample solution into the checking space 4 becomes weak. For this reason, when the sample solution is not sucked into the checking space 4, the discoloring agent 6 does not change color, or even if the sample solution is sucked, the sample solution is not sufficiently mixed with the discoloring agent 6 and color unevenness occurs. Further, when air remains in the tube of the needle N, the air is also sucked into the checking space 4 together with the sample solution, by inserting the distal end portion of the needle N into the checking space 4. Therefore, air is discharged from the needle N.

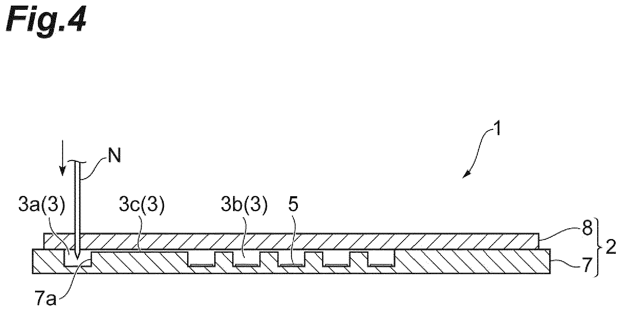

FIG. 4 is a schematic cross-sectional view illustrating a state in which the sample solution is punctured and injected into the reaction space. As illustrated in FIG. 4, in the reaction step (S2), the sample solution is punctured and injected into the injection space 3a, using the same needle N as in the checking step (S1). Specifically, the needle N is punctured into the second plate-shaped part 8, and the distal end portion of the needle N is inserted into the injection space 3a.

Then, when the depressurized state of the reaction space 3 is sufficiently maintained, the sample solution is sucked from the needle N to the injection space 3a by the negative pressure of the reaction space 3, and spreads to the overall reagent sealing space 3b from the injection space 3a via the flow path 3c. Further, the reagent 5 is mixed with the sample solution in each reagent sealing space 3b. Thereafter, the nucleic acid is amplified by incubation at a predetermined temperature.

Thereafter, presence or absence of an amplification product is detected in each reagent sealing space 3b. For example, a fluorescently labeled probe that specifically binds to the amplification product is previously contained in the reagent, and the amplification product is detected by irradiating each reagent sealing space 3b with excitation beam using a reflection method, a transmission method, or the like. The reflection method is a method of detecting the amplification product, by irradiating each reagent sealing space 3b with excitation beam and detecting fluorescence by a detection unit located on the same side as a light source with respect to the microchip 1. The transmission method is a method of detecting the amplification product, by irradiating each reagent sealing space 3b with excitation beam, and detecting fluorescence by a detection unit located on an opposite side to the light source with respect to the microchip 1.

Further, it is also possible to detect an amplification product, not only after the amplification reaction, but also by observing the amplification reaction over time. Furthermore, it is also possible to perform the multiple nucleic acid amplification reactions, by making the primer sets sealed in each reagent sealing space 3b different from each other.

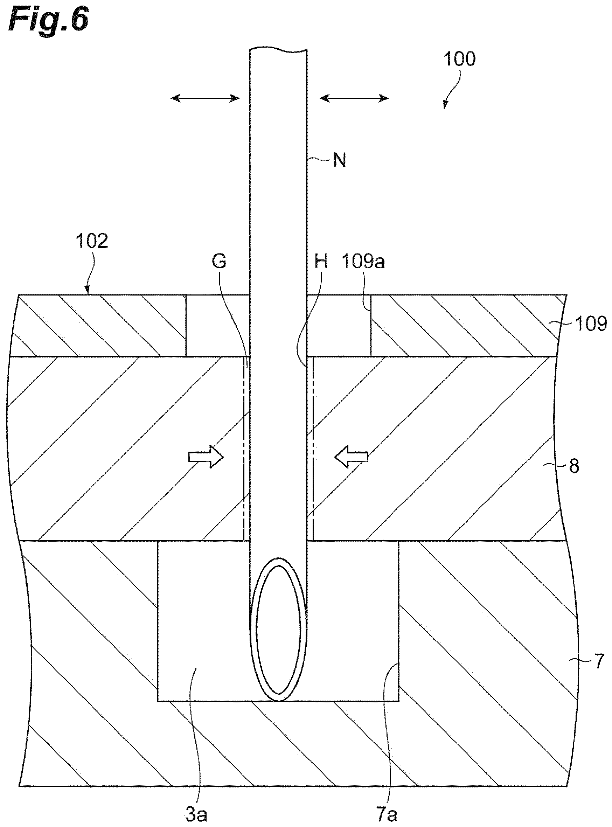

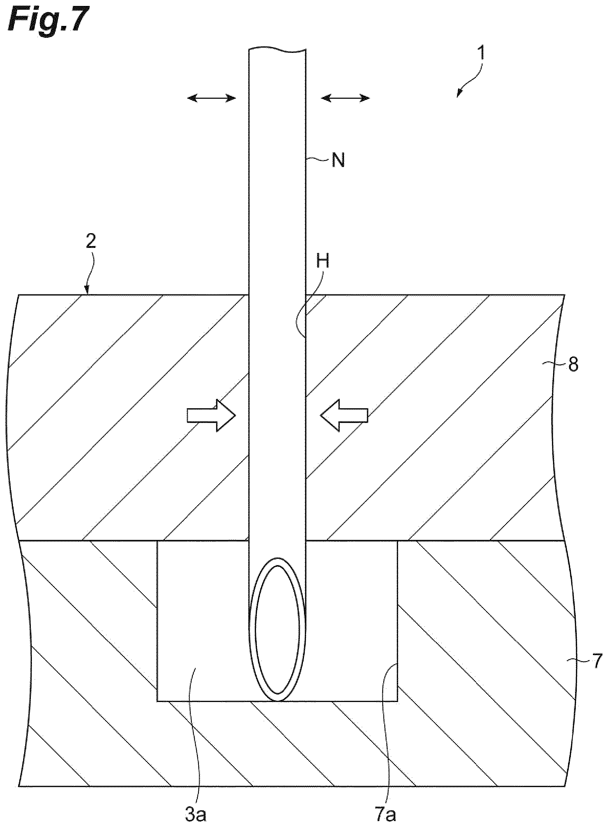

Here, the operation of the microchip 1 of the present example will be described in comparison with a microchip of a comparative example in which the chip main body has a three-layer structure. FIG. 6 is a schematic cross-sectional view illustrating a state in which the needle is punctured in the microchip of the comparative example. FIG. 7 is a schematic cross-sectional view illustrating a state in which the needle is punctured in the microchip of the non-claimed example. Further, FIGS. 6 and 7 illustrate a state in which the needle N is punctured in the second plate-shaped part 8, and the distal end portion of the needle N is inserted into the injection space 3a of the reaction space 3.

As illustrated in FIG. 6, a microchip 100 of the comparative example is the same as the microchip 1 of the present example except that a chip main body has a three-layer structure. Specifically, in the microchip 100 of the comparative example, a chip main body 102 has a three-layer structure of a first plate-shaped part 7, a second plate-shaped part 8 and a third plate-shaped part 109.

The third plate-shaped part 109 is formed in a substantially thin rectangular plate shape. The third plate-shaped part 109 is stacked on the side of the second plate-shaped part 8 opposite to the first plate-shaped part 7 to face the first plate-shaped part 7. Like the first plate-shaped part 7, the third plate-shaped part 109 has gas impermeability. The same material as the first plate-shaped part 7 can be used as the material of the third plate-shaped part 109. In the third plate-shaped part 109, a penetration hole 109a through which the needle N penetrates is formed at a position corresponding to each of the injection space 3a and the checking space 4.

In the microchip 100 thus configured, since the chip main body 102 has a three-layer structure of the first plate-shaped part 7, the second plate-shaped part 8 and the third plate-shaped part 109, when the overall thickness is constant, it is necessary to make the second plate-shaped part 8 thinner by the thickness of the third plate-shaped part 109. For example, when the thickness of the entire microchip 100 is 4.0 mm, the thickness of the first plate-shaped part 7 is 2.1 mm, the thickness of the second plate-shaped part 8 is 1.4 mm, and the thickness of the third plate-shaped part 109 is 0.5 mm.

When the second plate-shaped part 8 becomes thin, since the force with which the second plate-shaped part 8 presses the needle N weakens, the needle N punctured in the second plate-shaped part 8 easily deflects. Therefore, there is a possibility that a gap G is generated between the needle N and the second plate-shaped part 8, and air flows into the reaction space from the gap G. Moreover, there is also a possibility that a crack may extend from the puncture hole H formed by the puncturing of the needle N, and air may flow into the reaction space from the extended crack.

In contrast, in the microchip 1 of the present non-claimed example, the chip main body 2 has a two-layer structure of the first plate-shaped part 7 and the second plate-shaped part 8. Therefore, when the entire thickness is constant, the second plate-shaped part 8 can be made thicker than the microchip 100 of the comparative example. For example, when the thickness of the entire microchip 1 is 4.0 mm, the thickness of the first plate-shaped part 7 is 2.1 mm, and the thickness of the second plate-shaped part 8 is 1.9 mm. Thus, the thickness of the second plate-shaped part 8 can be increased by 0.5 mm as compared to the microchip 100 of the comparative example.

For this reason, as illustrated in FIG. 7, since the force with which the second plate-shaped part 8 presses the needle N becomes stronger than that of the microchip 100 of the comparative example, the deflection of the needle punctured in the second plate-shaped part is suppressed. As a result, it is possible to suppress an occurrence of a gap between the needle N and the second plate-shaped part 8, and also to suppress the extension of the crack from the puncture hole H to be formed by the puncturing of the needle N.

In this way, in the microchip 1 of the present non-claimed example, since the chip main body 2 has a two-layer structure of the first plate-shaped part 7 and the second plate-shaped part 8, the second plate-shaped part 8 can be made thicker, while suppressing the overall thickness of the microchip 1, as compared to the microchip 100 of the comparative example in which the chip main body 2 has a three-layer structure. As a result, since the pressing force of the needle N due to the second plate-shaped part 8 increases, the deflection of the needle N punctured in the second plate-shaped part 8 is suppressed. For this reason, it is possible to suppress the inflow of the air by puncturing of the needle N, while suppressing the heat distribution becoming uneven.

### [First Embodiment]

Next, a first embodiment will be described. The first embodiment is basically the same as the first example, and differs from the first example only in that a reinforcing film is stuck to the chip main body. Therefore, hereinafter, only the differences from the first example will be described, and the descriptions of the same matters as the first example will not be provided.

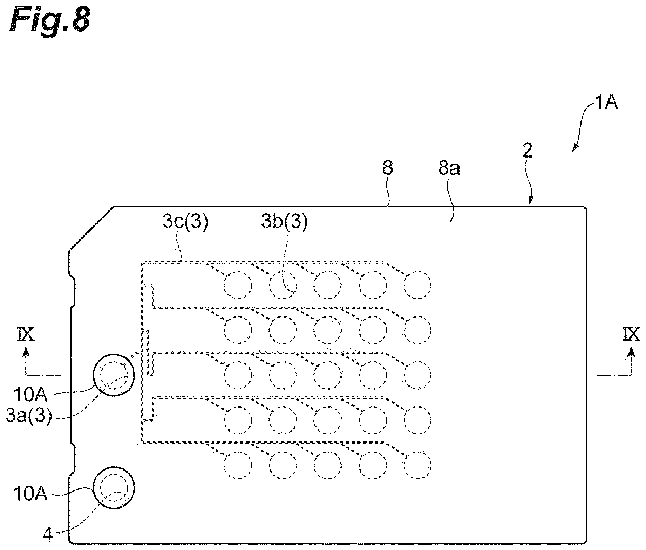

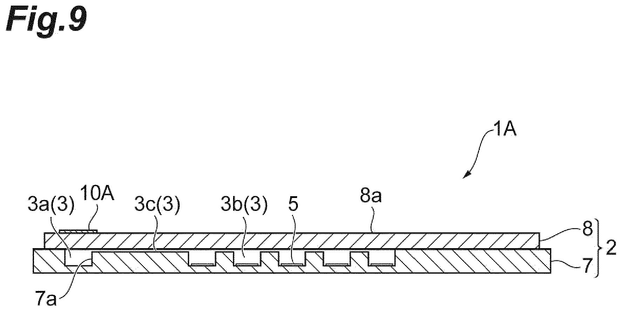

FIG. 8 is a plan view illustrating a microchip of the first embodiment. FIG. 9 is a schematic cross-sectional view taken along line IX-IX illustrated in FIG. 8. As illustrated in FIGS. 8 and 9, a microchip 1A of the first embodiment includes a chip main body 2 and a reinforcing film 10A.

The reinforcing film 10A is stuck to a surface 8a of the second plate-shaped part 8 on the side opposite to the first plate-shaped part 7. The reinforcing film 10A is a resin member having transparency, which is formed in a thin film shape or a tape shape.

The reinforcing film 10A reinforces the surface 8a of the second plate-shaped part 8 to suppress the deformation of the surface 8a of the second plate-shaped part 8 (to maintain the shape). Specifically, in the second plate-shaped part 8, the deflection of the needle N punctured in the second plate-shaped part 8 is the largest on the surface 8a. Therefore, the reinforcing film 10A suppresses the deflection of the needle N, by pressing the needle N radially inward, on the surface 8a on which the deflection of the needle N punctured in the second plate-shaped part 8 becomes largest. Therefore, it is preferable that the reinforcing film 10A be a film excellent in stretchability or a film excellent in shape retention.

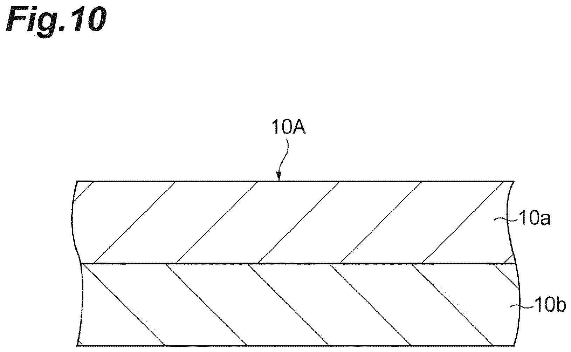

FIG. 10 is a schematic cross-sectional view of the reinforcing film. As illustrated in FIGS. 8 to 10, the reinforcing film 10A includes a base material 10a forming a main body of the reinforcing film 10A, and an adhesive part 10b stuck to the second plate-shaped part 8. The materials and thicknesses of the base material 10a and the adhesive part 10b can be set to, for example, the following first condition and second condition. However, the materials and thicknesses of the base material 10a and the adhesive part 10b are not limited to the following conditions.

The first condition is that the material of the base material 10a is polyurethane or polyester (PET), and the material of the adhesive part 10b is an acrylic adhesive or a silicone-based adhesive. Moreover, the thickness of the base material 10a is set to 10 µm or more and 30 µm or less, and the thickness of the adhesive part 10b is set to 20 µm or more and 120 µm or less. By setting the materials and thicknesses of the base material 10a and the adhesive part 10b to the first condition, the reinforcing film 10A having excellent elasticity is obtained.

The second condition is that the material of the base material 10a is polypropylene or polyester (PET), and the material of the adhesive part 10b is a silicone-based adhesive. Moreover, the thickness of the base material 10a is set to 20 µm or more and 100 µm or less, and the thickness of the adhesive part 10b is set to 20 µm or more and 100 µm or less. By setting the materials and thicknesses of the base material 10a and the adhesive part 10b to the second condition, the reinforcing film 10A having excellent shape retention can be obtained.

The reinforcing film 10A is stuck to a position facing the injection space 3a and a position facing the checking space 4, in the surface 8a of the second plate-shaped part 8, respectively. That is, the reinforcing film 10A is stuck to a position at which the needle N is punctured. The reinforcing film 10A is preferably formed in a circular shape that surrounds the position facing the injection space 3a and the checking space 4, respectively, but the shape is not particularly limited.

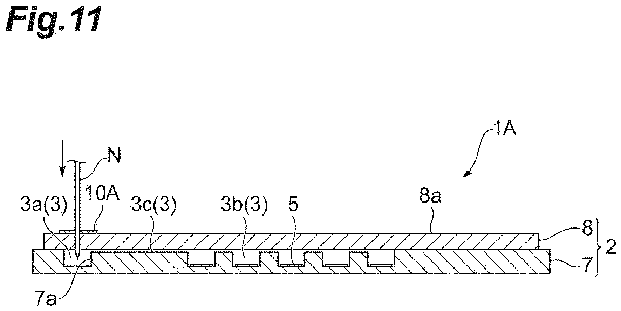

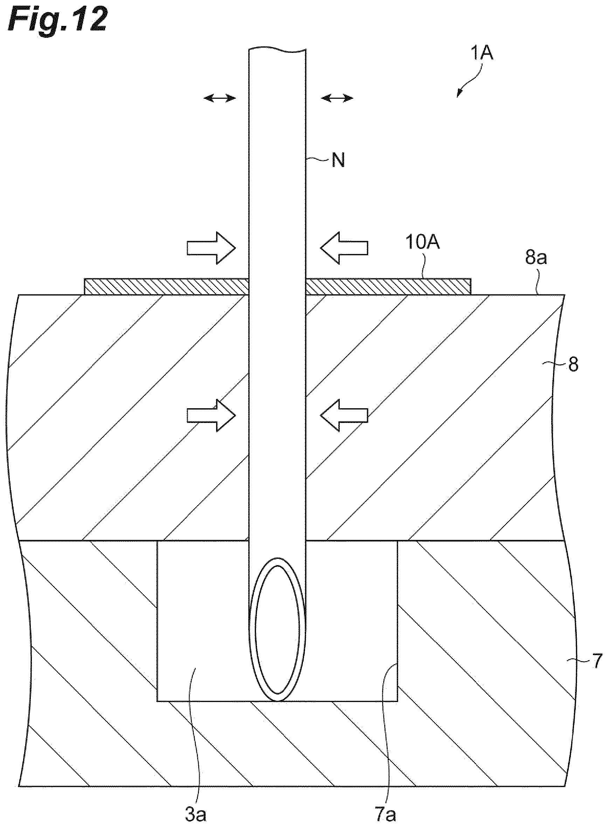

Next, a method of genetic test using the microchip 1A will be described with reference to FIGS. 11 and 12. FIG. 11 is a schematic cross-sectional view illustrating a state in which the sample solution is punctured and injected into the reaction space. FIG. 12 is a schematic cross-sectional view illustrating a state in which the needle is punctured in the microchip of the first embodiment. FIGS. 11 and 12 illustrate a state in which the needle N is punctured in the second plate-shaped part 8, and the distal end portion of the needle N is inserted into the injection space 3a of the reaction space 3.

In the present embodiment, as in the first example, the checking step (S1) and the reaction step (S2) are performed. In each of these steps, as illustrated in FIG. 11, the needle N is punctured at the position of the second plate-shaped part 8 to which the reinforcing film 10A is stuck, and the distal end portion of the needle N is inserted into the injection space 3a or the checking space 4.

At this time, as illustrated in FIG. 12, the needle N is pressed by the second plate-shaped part 8, and also pressed by the reinforcing film 10A. Moreover, since the reinforcing film 10A is stuck to the surface 8a of the second plate-shaped part 8 around the puncture hole H, extension of the crack from the puncture hole H is also suppressed.

Further, the sample solution sucked from the injection space 3a is mixed with the reagent 5 in each reagent sealing space 3b, and nucleic acid is amplified by incubation at a predetermined temperature.

In this way, in the microchip 1A of the present embodiment, since the reinforcing film 10A is stuck to the surface 8a of the second plate-shaped part 8, deformation of the second plate-shaped part 8 is suppressed at the position to which the reinforcing film 10A is stuck. Therefore, the deflection of the needle N punctured in the second plate-shaped part 8 can be suppressed, and the extension of the crack from the puncture hole H can be suppressed. This makes it possible to further suppress the inflow of air due to the puncturing of the needle N.

Further, in this microchip 1A, since the reinforcing film 10A is stuck to the position facing the injection space 3a and the position facing the checking space 4, respectively, when the needle is punctured, it is possible to efficiently suppress air from flowing in the reaction space 3 and the checking space 4.

Further, in the microchip 1A, high stretchability can be obtained by setting the material and thickness of the reinforcing film 10A as the first condition. For this reason, it is possible to suppress the puncture hole generated in the second plate-shaped part 8 from spreading, and to suppress extension of the crack from the puncture hole.

Further, in the microchip 1A, by setting the material and thickness of the reinforcing film 10A as the second condition, appropriate rigidity can be obtained. For this reason, the workability of sticking to the second plate-shaped part is improved. Furthermore, it is possible to suppress the puncture hole generated in the second plate-shaped part from spreading, and to suppress extension of the crack from the puncture hole.

### [Second Embodiment]

Next, a second embodiment will be described. The second embodiment is basically the same as the first embodiment, and only the shape of the reinforcing film is different from that of the first embodiment. Therefore, hereinafter, only differences from the first embodiment will be described, and the descriptions of the same matters as the first embodiment will not be provided.

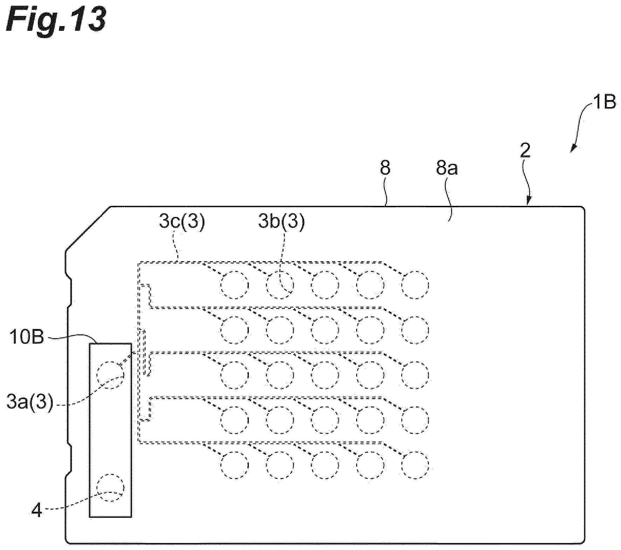

FIG. 13 is a plan view illustrating a microchip of the second embodiment. As illustrated in FIG. 13, a microchip 1B of the second embodiment includes a chip main body 2 and a reinforcing film 10B.

The reinforcing film 10B is integrally stuck to a position facing the injection space 3a and a position facing the checking space 4, in the surface 8a of the second plate-shaped part 8. That is, in the microchip 1B, one reinforcing film 10B is stuck to the surface 8a of the second plate-shaped part 8 to cover the position facing the injection space 3a and the position facing the checking space 4. The reinforcing film 10B is preferably formed in a rectangular shape or an elliptical shape that surrounds the position facing the injection space 3a and the checking space 4, but the shape is not particularly limited. The configuration, material and thickness of the reinforcing film 10B are the same as those of the reinforcing film 10A of the first embodiment.

As described above, in the microchip 1B of the second embodiment, since the reinforcing film 10B is integrally stuck to the position facing the injection space 3a and the position facing the checking space 4, workability at the time of sticking the reinforcing film 10B to both positions is improved.

### [Third Embodiment]

Next, a third embodiment will be described. The third embodiment is basically the same as the first embodiment, and only the shape of the reinforcing film is different from that of the first embodiment. Therefore, hereinafter, only differences from the first embodiment will be described, and the descriptions of the same matters as the first embodiment will not be provided.

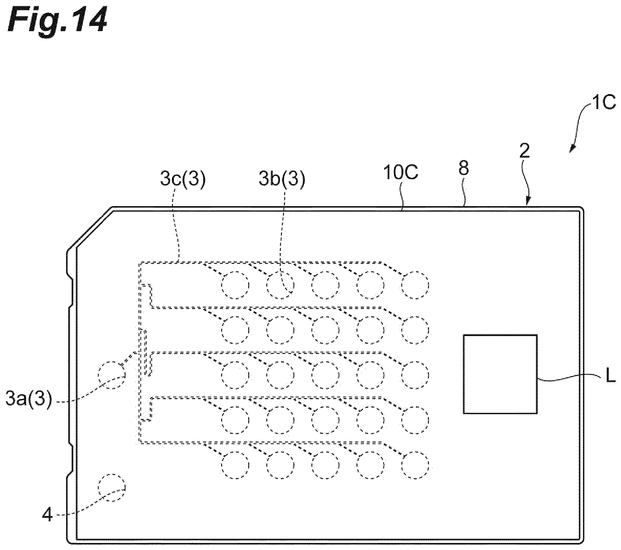

FIG. 14 is a plan view illustrating a microchip of the third embodiment. As illustrated in FIG. 14, a microchip 1C of the third embodiment includes a chip main body 2 and a reinforcing film 10C.

The reinforcing film 10C is stuck to the entire surface 8a of the second plate-shaped part 8. That is, the reinforcing film 10C is also stuck to a surplus position which does not face the reaction space 3 and the checking space 4, in the surface 8a of the second plate-shaped part 8, other than the position facing the reaction space 3 (the injection space 3a, the reagent sealing space 3b, and the flow path 3c) and the checking space 4. Further, the reinforcing film 10C may not be stuck to the entire surface 8a of the second plate-shaped part 8, and may be stuck to a region of 50% or more, and further 75% or more of the surface 8a of the second plate-shaped part 8. The configuration, the material, and the thickness of the reinforcing film 10C are the same as those of the reinforcing film 10A of the first embodiment.

A character or code L indicating identification information or the like of the microchip 1C can be printed on the reinforcing film 10C. The character or code L are preferably printed at surplus positions that do not face the reaction space 3 and the checking space 4, but may be printed at positions facing the reaction space 3 and the checking space 4. In addition, the character or code L may be printed at any position of the reinforcing film 10C, or may be printed on the entire reinforcing film 10C.

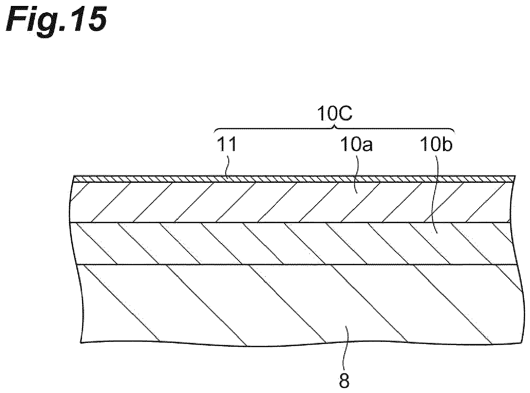

Further, from the viewpoint of suppressing stray light when detecting an amplification product, as illustrated in FIG. 15, the reinforcing film 10C preferably has a colored layer 11 in which the second plate-shaped part 8 side is colored in dark color such as black.

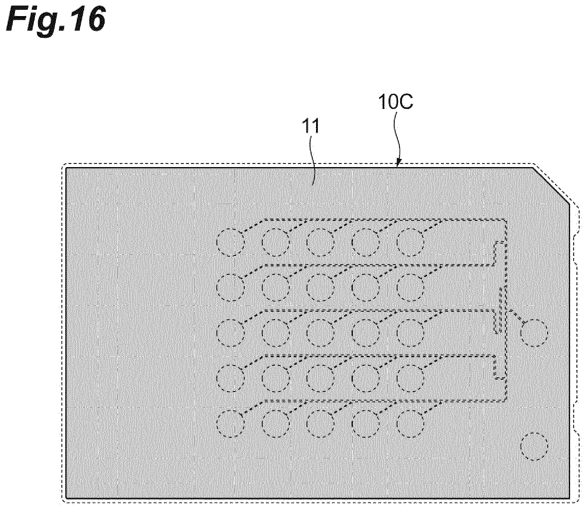

Further, when using a reflection method for detection of amplification of nucleic acid, as illustrated in FIG. 16, it is preferable that the colored layer be colored in dark color such as black on the entire surface of the side of the second plate-shaped part 8. Therefore, since the stray light of the light irradiated to each reagent sealing space 3b can be suppressed, detection accuracy can be enhanced. In this case, the character or code L may be printed on the entire surface or a part of the surface of the reinforcing film 10C on the side opposite to the second plate-shaped part 8.

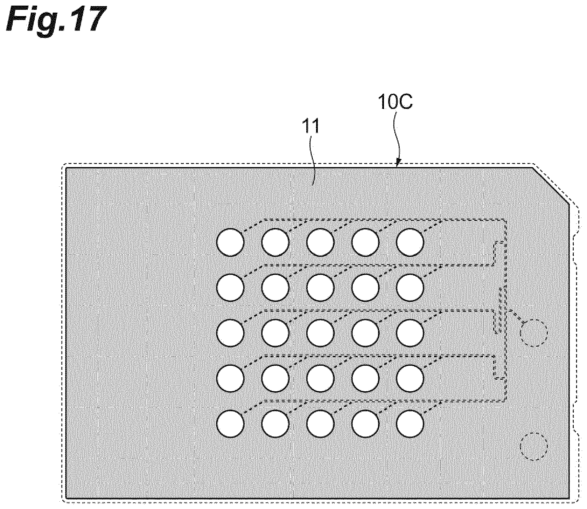

On the other hand, when the transmission method is used for detection of amplification of nucleic acid, as illustrated in FIG. 17, it is preferable that the filled colored layer be colored in a dark color such as black only at a position not facing the reagent sealing space 3b, in the surface of the second plate-shaped part 8 side. In addition, the position facing the reagent sealing space 3b is transparent. Thus, since it is possible to transmit the light irradiated to each reagent sealing space 3b and to suppress the stray light of the light irradiated to each reagent sealing space 3b, the detection accuracy can be enhanced. In this case, the character or code L may be printed on the entire surface or a part of the surface of the reinforcing film 10C, on the side opposite to the second plate-shaped part 8 of the portion colored in dark color such as black.

In this way, in the microchip 1C of the present embodiment, since the reinforcing film 10C is stuck to the entire surface 8a of the second plate-shaped part 8 or 50% or more of the surface 8a, it is possible to suppress the air from transmitting from the second plate-shaped part 8 side to the reaction space 3 and the checking space 4. Moreover, since the reinforcing film 10C is also stuck to the surplus position not facing the reaction space 3 and the checking space 4 of the second plate-shaped part 8, the character or code L can be printed on the portion of the reinforcing film 10C stuck to the surplus position. This makes it possible to reduce the work of sticking a label to the microchip 1C separately.

Further, in the microchip 1C, since the colored layer 11 is formed on the reinforcing film 10C, it is possible to suppress stray light when detecting the amplification of a nucleic acid.

As mentioned above, although embodiments of this invention were described, the invention is not limited to each embodiment. For example, the present invention may be applied to a microchip used for tests other than genetic tests that amplify nucleic acids, and the chip main body may also be appropriately changed in configuration. Further, the checking space may not be formed in the chip main body.

### Reference Signs List

1, 1A, 1B, 1C: microchip, 2: chip main body, 3: reaction space, 3a: injection space, 3b: reagent sealing space, 3c: flow path, 4: checking space, 5: reagent, 6: discoloring agent, 7: first plate-shaped part, 7a: reaction space recessed part, 7b: checking space recessed part, 8: second plate-shaped part, 8a: surface, 10A, 10B, 10C: reinforcing film, 10a: base material, 10b: adhesive part, 100: microchip, 102: chip main body, 109: third plate-shaped part, 109a: penetration hole, G: gap, H: puncture hole, L: character or code, N: needle.

## Claims

1. A microchip (1, 1A, 1B, 1C) equipped with a chip main body (2) in which a reaction space (3) depressurized with respect to an atmospheric pressure is formed,
wherein the chip main body (2) has a two-layer structure of a first plate-shaped part (7) having gas impermeability, and a second plate-shaped part (8) having a self-sealing property, which is laminated on one surface of the first plate-shaped part (7), and
the reaction space is formed between the first plate-shaped part (7) and the second plate-shaped part (8),
wherein the microchip (1, 1A, 1B, 1C) further comprises a reinforcing film (10A, 10B, 10C) stuck to a surface of the second plate-shaped part (8) on a side opposite to the first plate-shaped part (7),
wherein the reaction space (3) has an injection space (3a) for puncturing and injecting a sample solution, and
the reinforcing film (10A, 10B, 10C) is stuck to a position facing the injection space (3a), wherein the second plate-shape part is made of an elastic material, which allows for self-sealing of any holes made by puncturing or the like due to its own elastic deformation.

2. The microchip (1, 1A, 1B, 1C) according to claim 1, wherein a checking space (4) independent of the reaction space (3) is formed between the first plate-shaped part (7) and the second plate-shaped part (8), and
the reinforcing film (10A, 10B, 10C) is stuck to a position facing the checking space (4) wherein the checking space is decompressed with respect to the atmospheric pressure and wherein a discoloring agent (6) suitable to change colour when the sample solution is mixed with it is sealed in the checking space.

3. The microchip (1, 1A, 1B, 1C) according to claim 2, wherein the reinforcing film (10A, 10B, 10C) is integrally stuck to the position facing the injection space (3a) and the position facing the checking space (4).

4. The microchip (1, 1A, 1B, 1C) according to any one of claims 1 to 3, wherein the reinforcing film (10A, 10B, 10C) is stuck to the entire surface of the second plate-shaped part (8) on the side opposite to the first plate-shaped part (7).

5. The microchip (1, 1A, 1B, 1C) according to any one of claims 1 to 3, wherein the reinforcing film (10A, 10B, 10C) is stuck to 50% or more of the surface of the second plate-shaped part (8) on the side opposite to the first plate-shaped part (7).

6. The microchip (1, 1A, 1B, 1C) according to any one of claims 1 to 5, wherein the reinforcing film (10A, 10B, 10C) includes a base material (10a), and an adhesive part (10b) for sticking the base material (10a) to the second plate-shaped part (8),
a material of the base material (10a) is polyurethane or polyester, and
a thickness of the base material (10a) is 10 µm or more and 30 µm or less.

7. The microchip (1, 1A, 1B, 1C) according to any one of claims 1 to 5, wherein the reinforcing film (10A, 10B, 10C) includes a base material (10a), and an adhesive part (10b) for sticking the base material (10a) to the second plate-shaped part (8),
a material of the base material (10a) is polypropylene or polyester, and
a thickness of the base material (10a) is 20 µm or more and 100 µm or less.

8. The microchip (1, 1A, 1B, 1C) according to any one of claims 1 to 7, wherein the reinforcing film (10A, 10B, 10C) has a colored layer (11) in which the side of the colored layer (11) that faces the second plate-shaped part (8) is colored in dark color.

## Patentansprüche

1. Mikrochip (1, 1A, 1B, 1C), der mit einem Chip-Hauptkörper (2) ausgestattet ist, in dem ein in Bezug auf einen atmosphärischen Druck druckloser Reaktionsraum (3) ausgebildet ist,
wobei der Chip-Hauptkörper (2) eine zweischichtige Struktur aus einem ersten plattenförmigen Teil (7) mit Gasundurchlässigkeit und einem zweiten plattenförmigen Teil (8) mit einer selbstdichtenden Eigenschaft aufweist, der auf eine Oberfläche des ersten plattenförmigen Teils (7) laminiert ist, und
der Reaktionsraum zwischen dem ersten plattenförmigen Teil (7) und dem zweiten plattenförmigen Teil (8) ausgebildet ist,
wobei der Mikrochip (1, 1A, 1B, 1C) ferner eine Verstärkungsfolie (10A, 10B, 10C) umfasst, die an einer Oberfläche des zweiten plattenförmigen Teils (8) auf einer dem ersten plattenförmigen Teil (7) entgegengesetzten Seite angeklebt ist,
wobei der Reaktionsraum (3) einen Injektionsraum (3a) zum Durchstechen und Injizieren einer Probenlösung aufweist, und
die Verstärkungsfolie (10A, 10B, 10C) an eine Position geklebt ist, die dem Injektionsraum (3a) zugewandt ist, wobei der zweite plattenförmige Teil aus einem elastischen Material hergestellt ist, das aufgrund seiner eigenen elastischen Verformung eine Selbstabdichtung von durch Punktion oder dergleichen entstandenen Löchern ermöglicht.

2. Mikrochip (1, 1A, 1B, 1C) nach Anspruch 1 , bei dem zwischen dem ersten plattenförmigen Teil (7) und dem zweiten plattenförmigen Teil (8) ein von dem Reaktionsraum (3) unabhängiger Prüfraum (4) ausgebildet ist, und
die Verstärkungsfolie (10A, 10B, 10C) an einer dem Prüfraum (4) zugewandten Position angeklebt ist, wobei der Prüfraum gegenüber dem Atmosphärendruck dekomprimiert ist und wobei ein Verfärbungsmittel (6), das geeignet ist, die Farbe zu ändern, wenn die Probenlösung damit vermischt wird, in dem Prüfraum eingeschlossen ist.

3. Mikrochip (1, 1A, 1B, 1C) nach Anspruch 2, bei dem die Verstärkungsfolie (10A, 10B, 10C) an der dem Injektionsraum (3a) zugewandten Stelle und an der dem Prüfraum (4) zugewandten Stelle einstückig aufgeklebt ist.

4. Mikrochip (1, 1A, 1B, 1C) nach einem der Ansprüche 1 bis 3, bei dem die Verstärkungsfolie (10A, 10B, 10C) vollflächig auf die dem ersten plattenförmigen Teil (7) gegenüberliegende Seite des zweiten plattenförmigen Teils (8) geklebt ist.

5. Mikrochip (1, 1A, 1B, 1C) nach einem der Ansprüche 1 bis 3, bei dem die Verstärkungsfolie (10A, 10B, 10C) auf 50% oder mehr der Oberfläche des zweiten plattenförmigen Teils (8) auf der dem ersten plattenförmigen Teil (7) gegenüberliegenden Seite aufgeklebt ist.

6. Mikrochip (1, 1A, 1B, 1C) nach einem der Ansprüche 1 bis 5, bei dem die Verstärkungsfolie (10A, 10B, 10C) ein Basismaterial (10a) und einen Klebstoffteil (10b) zum Ankleben des Basismaterials (10a) an den zweiten plattenförmigen Teil (8) aufweist,
ein Material des Basismaterials (10a) Polyurethan oder Polyester ist, und eine Dicke des Basismaterials (10a) 10 µm oder mehr und 30µm oder weniger beträgt.

7. Mikrochip (1, 1A, 1B, 1C) nach einem der Ansprüche 1 bis 5, bei dem die Verstärkungsfolie (10A, 10B, 10C) ein Basismaterial (10a) und einen Klebstoffteil (10b) zum Ankleben des Basismaterials (10a) an den zweiten plattenförmigen Teil (8) aufweist,
ein Material des Basismaterials (10a) Polypropylen oder Polyester ist, und
eine Dicke des Basismaterials (10a) 20 µm oder mehr und 100 µm oder weniger beträgt.

8. Mikrochip (1, 1A, 1B, 1C) nach einem der Ansprüche 1 bis 7, bei dem die Verstärkungsfolie (10A, 10B, 10C) eine farbige Schicht (11) aufweist, bei der die dem zweiten plattenförmigen Teil (8) zugewandte Seite der farbigen Schicht (11) dunkel gefärbt ist.

## Revendications

1. Micropuce (1, 1A, 1B, 1C) équipée d'un corps principal de puce (2) dans lequel un espace réactionnel (3) dépressurisé par rapport à une pression atmosphérique est formé,
dans laquelle le corps principal de puce (2) présente une structure à deux couches constituée d'une première partie en forme de plaque (7) ayant une imperméabilité aux gaz, et d'une seconde partie en forme de plaque (8) ayant une propriété d'auto-étanchéification, qui est stratifiée sur une surface de la première partie en forme de plaque (7), et
l'espace réactionnel est formé entre la première partie en forme de plaque (7) et la seconde partie en forme de plaque (8),
dans laquelle la micropuce (1, 1A, 1B, 1C) comprend en outre un film de renforcement (10A, 10B, 10C) collé à une surface de la seconde partie en forme de plaque (8) sur un côté opposé à la première partie en forme de plaque (7),
dans laquelle l'espace réactionnel (3) a un espace d'injection (3a) pour perforation et injection d'une solution d'échantillon, et
le film de renforcement (10A, 10B, 10C) est collé à une position faisant face à l'espace d'injection (3a), dans laquelle la seconde partie en forme de plaque est faite d'un matériau élastique, qui permet une auto-étanchéification de tous les trous réalisés par perforation ou analogue du fait de sa propre déformation élastique.

2. Micropuce (1, 1A, 1B, 1C) selon la revendication 1, dans laquelle un espace de vérification (4) indépendant de l'espace réactionnel (3) est formé entre la première partie en forme de plaque (7) et la seconde partie en forme de plaque (8), et
le film de renforcement (10A, 10B, 10C) est collé à une position faisant face à l'espace de vérification (4) dans laquelle l'espace de vérification est décompressé par rapport à la pression atmosphérique et dans laquelle un agent décolorant (6) approprié pour changer de couleur lorsque la solution d'échantillon est mélangée avec celui-ci, est renfermé dans l'espace de vérification.

3. Micropuce (1, 1A, 1B, 1C) selon la revendication 2, dans laquelle le film de renforcement (10A, 10B, 10C) est intégralement collé à la position faisant face à l'espace d'injection (3a) et à la position faisant face à l'espace de vérification (4).

4. Micropuce (1, 1A, 1B, 1C) selon l'une quelconque des revendications 1 à 3, dans laquelle le film de renforcement (10A, 10B, 10C) est collé à la surface entière de la seconde partie en forme de plaque (8) sur le côté opposé à la première partie en forme de plaque (7).

5. Micropuce (1, 1A, 1B, 1C) selon l'une quelconque des revendications 1 à 3, dans laquelle le film de renforcement (10A, 10B, 10C) est collé à 50 % ou plus de la surface de la seconde partie en forme de plaque (8) sur le côté opposé à la première partie en forme de plaque (7).

6. Micropuce (1, 1A, 1B, 1C) selon l'une quelconque des revendications 1 à 5, dans laquelle le film de renforcement (10A, 10B, 10C) comprend un matériau de base (10a), et une partie adhésive (10b) pour coller le matériau de base (10a) à la seconde partie en forme de plaque (8),
un matériau du matériau de base (10a) est du polyuréthane ou du polyester, et
une épaisseur du matériau de base (10a) est de 10 µm ou plus et de 30 µm ou moins.

7. Micropuce (1, 1A, 1B, 1C) selon l'une quelconque des revendications 1 à 5, dans laquelle le film de renforcement (10A, 10B, 10C) comprend un matériau de base (10a), et une partie adhésive (10b) pour coller le matériau de base (10a) à la seconde partie en forme de plaque (8),
un matériau du matériau de base (10a) est du polypropylène ou du polyester, et
une épaisseur du matériau de base (10a) est de 20 µm ou plus et de 100 µm ou moins.

8. Micropuce (1, 1A, 1B, 1C) selon l'une quelconque des revendications 1 à 7, dans laquelle le film de renforcement (10A, 10B, 10C) a une couche colorée (11) dans laquelle le côté de la couche colorée (11) qui fait face à la seconde partie en forme de plaque (8) est coloré en couleur foncée.
